# FASCICULE DE BREVET EUROPEEN

(11) **EP 1 234 546 B1**
(45) Date de publication et mention de la délivrance du brevet: **19.05.2004**
(21) Numéro de dépôt: 02356037.8
(22) Date de dépôt: 25.02.2002
(51) Int. Cl.: A61B 17/68

(54) **Dispositif de protection de broches chirurgicales**
Vorrichtung zum Schutz von chirurgischen Stiften
Protection device for surgical pins

(30) Priorité: 26.02.2001 FR 0102577
(43) Date de publication de la demande: 28.08.2002
(73) Titulaire: Newdeal S.A., 69006 Lyon (FR)
(72) Inventeur: Delmi, Marino, 1263 Vandoeuvres (CH)
(74) Mandataire: Martin, Didier Roland Valéry

(56) Documents cités:
- FR-A- 2 633 822
- FR-A- 2 734 470
- US-A- 5 300 072
- US-A- 5 330 476

## Description

La présente invention se rapporte au domaine technique général des broches chirurgicales destinées à assurer la solidarisation et l'alignement de deux parties osseuses, et en particulier de phalanges d'orteils ou de doigts, ladite solidarisation s'effectuant par mise en contact des deux parties osseuses.

La présente invention concerne un dispositif chirurgical destiné à assurer la solidarisation et l'alignement d'au moins deux parties osseuses entre elles, ledit dispositif comportant :
- une broche chirurgicale apte à être implantée axialement dans les parties osseuses solidarisées,
- un bouchon de protection pourvu d'un canal traversant pour permettre l'enfilement sur la broche, ledit bouchon comportant un orifice de blocage dans lequel un moyen de blocage est destiné à être introduit pour bloquer la position relative du bouchon et de la broche.

Dans diverses situations chirurgicales, et par exemple dans le cas de fractures ou de déformations osseuses des phalanges du pied ou de la main, il est nécessaire de procéder à la solidarisation de phalanges du pied ou de la main.

De manière générale, les chirurgiens ont alors recours à des éléments mécaniques externes conçus pour assurer, pendant une période déterminée, la solidarisation et le maintien en place des fractions osseuses à ostéosynthétiser.

Ainsi, on connaît déjà la possibilité d'avoir recours à l'utilisation d'agrafes à compression. Leur utilisation n'est pas d'application générale et s'avère souffrir d'inconvénients divers liés notamment à un maintien souvent insuffisamment stable des parties osseuses à solidariser. Les agrafes à compression souffrent également d'un inconvénient lié à la difficulté de leur mise en place, la recherche d'une direction axiale précise s'avérant souvent extrêmement difficile à réaliser, puis à maintenir.

C'est la raison pour laquelle on préfère souvent utiliser des montages faisant intervenir l'insertion percutanée d'une broche chirurgicale, qui est enfilée axialement à travers les fractions osseuses à solidariser.

Le recours à des broches métalliques, traversant tout ou partie des deux parties osseuses à solidariser, présente bien évidemment l'avantage de permettre un maintien des os selon une direction axiale particulièrement bien alignée. Généralement, une telle broche est mise en place à l'aide d'un moteur pour faciliter la pénétration dans l'os de l'extrémité libre de la broche, l'extrémité libre non pénétrante, étant alors obligatoirement présente au niveau percutané du pied ou de la main. Cette extrémité libre peut présenter un certain danger et est en conséquence généralement recourbée par le chirurgien.

Pour diminuer les risques d'accrochages, voire de blessures, il existe également des protections métalliques ou plastiques formant une sorte de bouchon qui s'enfile sur la broche à partir de l'extrémité libre par un canal traversant ménagé à travers la masse du bouchon.

Les dispositifs de ce type actuellement connus comprennent un bouchon de protection sphérique traversé selon son axe de symétrie par un canai de section cylindrique (voir, par exemple le document FR 2,633,822; le préambule de la revendication 1 est basé sur ce document). Un tel dispositif connu est également pourvu d'un orifice de blocage taraudé débouchant dans le canal traversant et dirigé selon une direction sensiblement orthogonale à ce dernier. L'orifice de blocage comporte intérieurement une vis simple déplaçable formant un moyen de blocage de la position relative du bouchon et de la broche. En effet, lorsque la broche est en place, et que le bouchon est enfilé par le biais du canal traversant, le bouchon peut être bloqué en position grâce à la vis de blocage qui, par vissage, vient comprimer la broche au niveau de la jonction entre le canal traversant et l'orifice de blocage.

Ce dispositif permet en conséquence une certaine protection de l'extrémité libre de la broche et évite au patient de se blesser ou de s'accrocher à différents obstacles au cours des gestes de sa vie quotidienne. Lorsque la fusion osseuse est réalisée, le chirurgien procède alors au retrait de la broche en s'aidant d'une pince de préhension qui permet l'extraction axiale et la rotation nécessaire au glissement de la broche. Si les dispositifs connus à ce jour apportent une contribution positive à la protection de l'embout de broche, ils s'avèrent peu efficaces pour toutes les manipulations requises pour l'extraction de la broche, ce qui implique nécessairement le recours à un outil additionnel d'aide à l'extraction.

Un tel dispositif connu apporte bien évidemment une contribution positive aux protections des patients, mais souffre néanmoins de divers inconvénients, et notamment de la nécessité d'avoir recours à un filetage interne à l'orifice de blocage. Une telle opération est en effet délicate à réaliser sur des éléments aussi petits que les bouchons de protections utilisés, ce qui rend l'opération industrielle de filetage délicate à réaliser. Par ailleurs, une telle opération implique un surcoût industriel non négligeable, d'autant qu'une telle opération ne peut être réalisée qu'avec une précision importante et avec un matériel correspondant onéreux.

Par ailleurs, la réalisation d'un tel dispositif s'avère poser des problèmes additionnels de démoulage de pièces, ce qui, en définitive, conduit à limiter l'emploi de ces dispositifs connus.

Les objets assignés à l'invention visent en conséquence à porter remède aux divers inconvénients énumérés précédemment et à proposer un nouveau dispositif chirurgical destiné à assurer la solidarisation et l'alignement de parties osseuses entre elles, et en particulier de phalanges de pieds ou de mains, un tel dispositif étant particulièrement simple à réaliser et à utiliser, notamment en facilitant son extraction, tout en étant d'un coût réduit.

Un autre objet de l'invention vise à proposer un nouveau dispositif chirurgical permettant de réduire les risques de blessure ou d'accrochage par le patient.

Un autre objet de l'invention vise à proposer un nouveau dispositif chirurgical permettant d'assurer de manière particulièrement simple et fiable le blocage d'un bouchon de protection sur une broche chirurgicale.

Un autre objet de l'invention vise à proposer un nouveau dispositif chirurgical permettant de s'adapter avec un même bouchon à différents diamètres de broches chirurgicales.

Les objets assignés à l'invention sont atteints à l'aide d'un dispositif chirurgical destiné à assurer la solidarisation et l'alignement d'au moins deux parties osseuses entre elles, comportant :
- une broche chirurgicale apte à être implantée axialement dans les parties osseuses à solidariser,
- un bouchon de protection pourvu d'un canal traversant pour permettre l'enfilement sur la broche, ledit bouchon comportant un orifice de blocage dans lequel un moyen de blocage est destiné à être introduit pour bloquer la position relative du bouchon et de la broche,
caractérisé en ce que :
- l'orifice de blocage est ménagé de manière à déboucher sensiblement tangentiellement dans le canal traversant pour former une surface S,
- le moyen de blocage présentant une section transversale adaptée de manière à ce que, lorsqu'il est introduit dans l'orifice de blocage, il puisse bloquer relativement le bouchon et la broche par contact compressif / bloquant au niveau de la surface S.

L'invention sera mieux comprise à la lecture de la description qui suit et des dessins annexés, fournis à titre purement explicatif et non limitatif dans lesquels :
- La figure 1 illustre, selon une vue en perspective, un dispositif chirurgical correspondant à l'invention et comportant une broche chirurgicale et un bouchon de protection avant l'enfilement du moyen de blocage.
- La figure 2 illustre, selon une vue en perspective correspondant à la figure 1, un dispositif chirurgical conforme à l'invention avec un moyen de blocage avant rupture de sa tête.
- La figure 3 illustre, selon une vue analogue aux figures 1 et 2, un dispositif chirurgical conforme à l'invention après rupture de la tête du moyen de blocage.
- La figure 4 illustre, selon une vue latérale en perspective, un détail de réalisation du moyen de blocage et du bouchon de protection.
- La figure 5 illustre, selon une vue en perspective, un détail de réalisation du moyen de blocage avant sa rupture.
- La figure 6 illustre, selon une vue en coupe transversale, un détail de réalisation du corps du moyen de blocage.
- La figure 7 illustre, selon une vue en coupe transversale identique à celle de la figure 6, un détail de réalisation d'une variante préférentielle du corps du moyen de blocage.
- La figure 8 illustre, selon une vue en perspective, une variante préférentielle de réalisation.

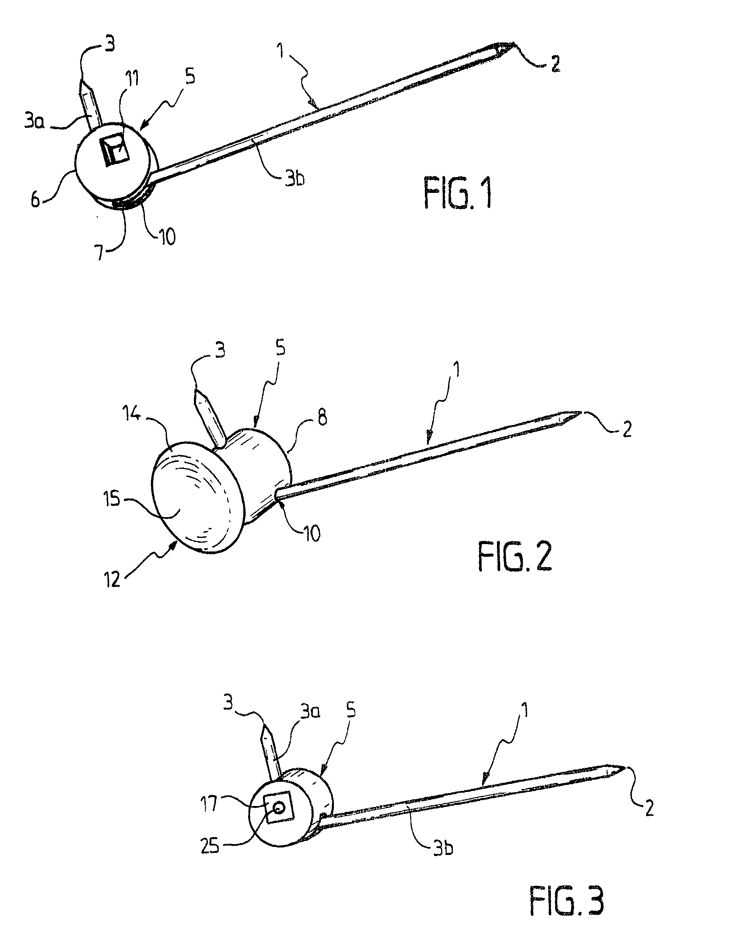

Le dispositif chirurgical illustré aux figures 1 à 8 est destiné à assurer la solidarisation et l'alignement d'au moins deux parties osseuses entre elles (non représentées aux figures), de manière à pouvoir favoriser l'ostéosynthèse. Dans la description qui suit, il sera plus particulièrement fait référence à la solidarisation d'os des phalanges du pied ou de la main, étant entendu que le dispositif chirurgical conforme à l'invention ne saurait en aucune manière être limité à la solidarisation de ces types d'os, une telle application n'étant que préférentielle.

Le dispositif chirurgical conforme à l'invention comporte une broche chirurgicale 1 apte à être implantée axialement dans les parties osseuses à solidariser. A cette fin, la broche chirurgicale 1 se présente sous la forme d'une tige présentant une bonne résistance mécanique, et par exemple réalisée en matériau métallique à usage médical, du genre acier inoxydable ou titane par exemple. La broche chirurgicale 1 présente une forme générale cylindrique effilée, de quelques millimètres d'épaisseur et de quelques centimètres de longueur, pourvue à ses deux extrémités 2, 3, d'un biseau ou d'une extrémité pointue favorisant le perçage ou la pénétration dans les os à ostéosynthétiser. Avant son utilisation, la broche chirurgicale est rectiligne, mais présente une résistance mécanique appropriée permettant au chirurgien d'assurer facilement son pliage sans rupture.

Le dispositif chirurgical conforme à l'invention comporte également un bouchon de protection 5 réalisé par exemple en un matériau plastique à usage médical, de préférence susceptible d'être injecté, du genre polycarbonate ou polypropylène, se présentant par exemple sous la forme générale d'un corps de révolution sensiblement cylindrique définissant deux faces opposées 6, 7, sensiblement planes, réunies par une enveloppe cylindrique 8. Le bouchon de protection 5 est pourvu d'un canal traversant 9 ménagé dans la masse même du corps du bouchon de protection 5 pour former un canal rectiligne s'étendant entre deux points quelconques de l'enveloppe cylindrique 8. Le canal traversant 9 est avantageusement de diamètre constant et par exemple sensiblement supérieur aux diamètres usuels des broches chirurgicales connues, de manière à pouvoir assurer un passage facile de la broche chirurgicale 1 à travers ledit canal traversant 9.

Selon une version préférentielle de l'invention, tel qu'illustré aux figures 1 à 3 et 7, la broche chirurgicale 1 étant destinée à être coudée par le chirurgien lors de l'implantation, il est particulièrement avantageux que le bouchon de protection 5 comprenne également une rainure de guidage 10 pour la broche chirurgicale 1 et ménagée dans la masse du bouchon 5 et l'enveloppe cylindrique 8.

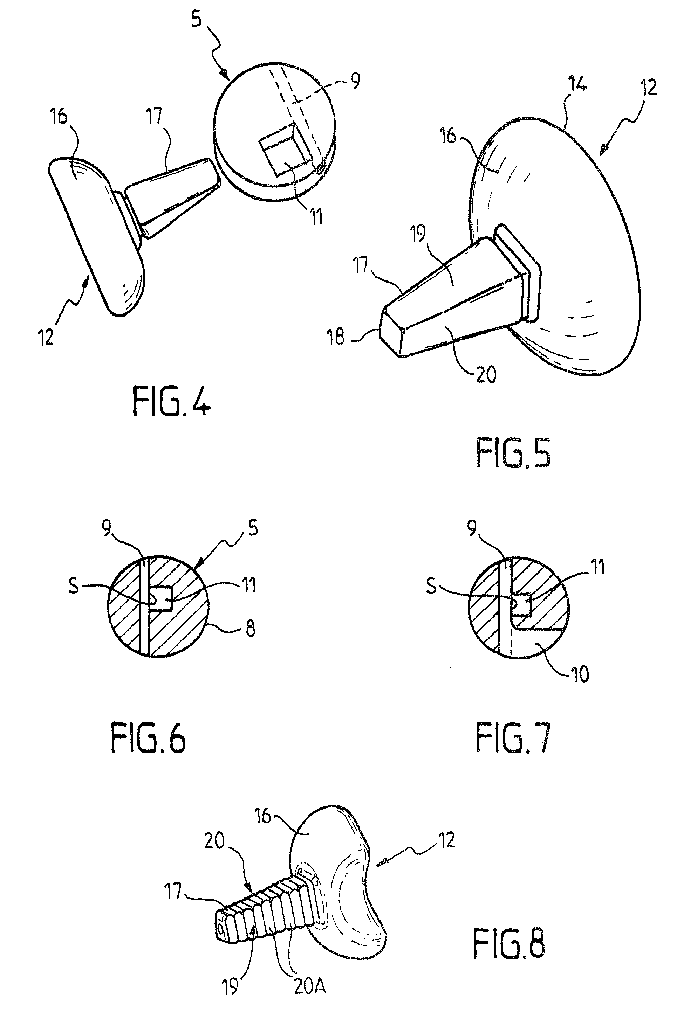

Tel qu'illustré à la figure 7, la rainure de guidage 10 s'étend à partir du canal traversant 9 et débouche dans ce dernier (ou vice versa), de préférence selon une direction perpendiculaire, pour former un coude à environ 90°. La rainure de guidage 10 s'étend jusqu'à l'enveloppe cylindrique 8 où elle débouche en formant un U avec deux flancs latéraux qui permettent de bloquer et de guider en leur sein la broche chirurgicale 1.

Tel qu'illustré aux figures 1 à 3, le canal traversant 9 s'étendant selon la direction illustrée par la branche 3a de la broche chirurgicale 1, la rainure de guidage 10 s'étend sensiblement perpendiculairement à la branche 3a au sein d'une fraction de l'enveloppe cylindrique 8 et selon la direction de la branche 3b à la sortie du canal traversant 9.

Le bouchon de protection 5 comporte également un orifice de blocage 11, de préférence traversant, ménagé dans la masse même du corps cylindrique 8 entre les deux faces 6, 7, et dans lequel un moyen de blocage 12 est destiné à être introduit pour bloquer la position relative du bouchon de protection 5 et de la broche chirurgicale 1.

Selon l'invention, l'orifice de blocage 11 est ménagé de manière à déboucher sensiblement tangentiellement dans le canal traversant 9 pour former une surface ou ouverture commune ou interface S, le moyen de blocage 12 présentant une section transversale adaptée, de manière que lorsqu'il est introduit dans l'orifice de blocage 11, il puisse bloquer relativement le bouchon de protection 5 et la broche chirurgicale 1 par contact compressif au niveau et par l'intermédiaire de ladite surface S. L'orifice de blocage 11 débouchant sur le côté et sur une fraction de longueur du canal traversant 9 permet, au moyen de blocage 12, de compresser la branche 3a de la broche chirurgicale 1 contre une portion de surface du canal traversant 9, ce qui assure son blocage et évite ainsi sa translation.

Grâce à ces caractéristiques, le bouchon 5 est ainsi bloqué axialement sur la broche 1, ce qui permet d'utiliser efficacement le bouchon 5 comme moyen de préhension pour extraire axialement la broche 1. Dans le cas où le bouchon 5 est également pourvu d'une rainure 10 de guidage pour couder la broche 1, la conjonction du coudage et du blocage axial renforce la stabilité en rotation du bouchon 5. Cette particularité renforce grandement la facilité d'extraction de la broche 1 puisque dans toutes les directions de manipulation lors de l'ablation ou retrait (actions combinées axiales et rotatoires), le bouchon 5 reste stable et solidaire de la broche 1.

Tel qu'illustré aux figures 1 à 7, l'orifice de blocage 11 est réalisé de manière à s'étendre selon une direction sensiblement orthogonale au canal traversant 9. Avantageusement, il présentera une section transversale sensiblement constante de forme et de dimensions adaptées au moyen de blocage 12. La section transversale de l'orifice de blocage 11 sera par exemple avantageusement sensiblement parallélépipédique, et par exemple rectangulaire ou carré, la section pouvant être néanmoins également circulaire, ovale ou quelconque.

Selon l'invention, le moyen de blocage 12 sera avantageusement réalisé sous la forme d'un élément poussoir 17 avec une tête de poussée 14, formant une coupelle avec une face de poussée 15, avantageusement concave. A partir de la face 16 opposée à la face de poussée 15, s'étend l'élément poussoir 17 proprement dit, destiné à être introduit dans l'orifice de blocage 11.

Selon l'invention, l'élément poussoir 17 présentera une section transversale de dimensions adaptées et de forme conjuguée à la section transversale de l'orifice de blocage 11, la section transversale de l'élément poussoir 17 étant de section croissante, et de préférence régulièrement croissante, à partir de son extrémité d'introduction 18 en direction de la face 16.

Tel qu'illustré aux figures 4 et 5 en particulier, la section transversale 11 étant de forme parallélépipédique, et par exemple carrée ou avantageusement rectangulaire, la section transversale de l'élément poussoir 17 sera également parallélépipédique, et respectivement carrée ou rectangulaire, tout en présentant une forme conique telle qu'illustrée.

Avantageusement, la section rectangulaire de l'élément poussoir 17 se prolonge donc suivant une génératrice conique.

Selon une version particulièrement avantageuse de l'invention, l'élément poussoir 17 présentera une section transversale rectangulaire se prolongeant suivant une génératrice conique, comme illustré aux figures 4, 5 et 8 permettant de définir une section de grande largeur et une section de petite largeur, respectivement matérialisées par deux faces de grande largeur 19, et deux faces de petite largeur 20.

Cette particularité permet, lors de l'introduction de l'élément poussoir 17, de choisir l'orientation de son introduction en fonction du diamètre de la broche chirurgicale 1 utilisée. Ainsi, si la broche chirurgicale 1 utilisée est d'un diamètre important, il faut orienter l'élément poussoir 17 dans l'orifice de blocage 11 en présentant sa section de plus faible largeur, de telle façon que l'une de ces faces 19 de grande largeur vienne en contact compressif contre la broche chirurgicale 1 au niveau de la surface S.

Inversement, si la broche utilisée est d'un diamètre plus faible, il faudra au contraire présenter la face de plus petite largeur 20, pour que celle-ci vienne en contact compressif par l'intermédiaire de la surface S avec la broche chirurgicale 1 de plus faible diamètre.

Dans les exemples de réalisation montrés aux figures 4 et 5, les surfaces des faces 19 et 20 sont planes. A titre de variante préférentielle (Figure 8), les faces 19 et 20 pourront également être pourvues de reliefs, et par exemple de crans 20A transversaux favorisant le blocage et empêchant la sortie hors de l'orifice de blocage 11.

Sur le plan pratique, on considère que le diamètre usuel des broches chirurgicales utilisées variant environ de 1 à 2 mm, il est possible d'assurer avec les faces 19 de plus grande largeur un blocage de broches chirurgicales d'un diamètre moyen de 1,6 à 2 mm, alors qu'avec les faces 20 de petite largeur il est possible d'assurer un blocage des broches chirurgicales d'un diamètre moyen de l'ordre de 1 à 1,6 mm.

De manière avantageuse, l'élément poussoir 17 étant surmonté de la tête 14, il est particulièrement utile de prévoir que le poussoir 12 soit sécable de manière à séparer la tête 14 de l'élément poussoir 17. Pour cela, et comme cela est bien connu de l'homme du métier, on prévoit entre la tête 12 et sa jonction avec l'élément poussoir 17 une zone de liaison 25 (Figure 3) de 0 faible section formant une zone de fragilisation aisément sécable.

En utilisation, le chirurgien, après avoir implanté la broche chirurgicale 1 rectiligne à travers les deux fractions d'os à solidariser, enfile un bouchon de protection 5 par l'extrémité 3 et par l'intermédiaire du canal traversant 9.

Le chirurgien positionne ensuite le bouchon de protection 5 à l'endroit désiré sur la portion de longueur libre de la broche chirurgicale 1, de telle sorte qu'une portion de longueur 3a de la broche chirurgicale 1 peut dépasser. Il est néanmoins envisageable, au sens de l'invention, de positionner le bouchon de protection 5 près de l'extrémité 3 de la broche chirurgicale 1, de telle sorte qu'aucune portion de la broche ne dépasse.

Dans un cas comme dans l'autre, le chirurgien saisit ensuite un moyen de blocage 12 conforme à l'invention par la tête 14, puis introduit l'élément poussoir 17 après l'avoir correctement orienté selon le diamètre de la broche 1 considérée dans l'orifice de blocage 11.

Par pression sur la face concave 15, le chirurgien introduit en force l'élément poussoir 17 jusqu'à venir bloquer la broche chirurgicale 1 dans le canal traversant 9 grâce au contact bloquant réalisé par l'une des faces 19, 20 de l'élément poussoir 17.

Le chirurgien peut ensuite procéder à la désolidarisation de la tête 14 par rupture au niveau de la zone de liaison sécable 25 (Figure 3), le bouchon de protection 5 étant désormais bloqué en position sur la broche chirurgicale 1.

Dans le cas où une portion de longueur 3a dépasse hors du bouchon de protection 5, le chirurgien peut d'une part sectionner, si nécessaire, le brin dépassant 3a, et d'autre part par pression sur ladite broche chirurgicale 1, réaliser un coude tel qu'illustré aux figures 1 à 3 par exemple.

## Revendications

1. Dispositif chirurgical destiné à assurer la solidarisation et l'alignement d'au moins deux parties osseuses entre elles, comportant :
- une broche chirurgicale (1) apte à être implantée axialement dans les parties osseuses à solidariser,
- un bouchon de protection (5) pourvu d'un canal traversant (9) pour permettre l'enfilement sur la broche (1), ledit bouchon comportant un orifice de blocage (11) dans lequel un moyen de blocage (12) est destiné à être introduit pour bloquer la position relative du bouchon (5) et de la broche (1),
**caractérisé en ce que** :
- l'orifice de blocage (11) est ménagé de manière à déboucher sensiblement tangentiellement dans le canal traversant (9) pour former une surface S,
- le moyen de blocage (12) présentant une section transversale adaptée de manière à ce que, lorsqu'il est introduit dans l'orifice de blocage (11), il puisse bloquer relativement le bouchon (5) et la broche (1) par contact compressif / bloquant au niveau de la surface S.

2. Dispositif selon la revendication 1 **caractérisé en ce que** l'orifice de blocage (11) est sensiblement orthogonal au canal traversant (9).

3. Dispositif selon l'une des revendications précédentes **caractérisé en ce que** le canal traversant (9) débouche dans une rainure de guidage (10), par exemple en U, ménagée dans la masse du bouchon (5) pour permettre de réaliser un coude d'arrêt sur la broche.

4. Dispositif selon l'une des revendications précédentes **caractérisé en ce que** le moyen de blocage (12) comprend un élément poussoir (17) de section transversale croissante à partir de son extrémité d'introduction (18).

5. Dispositif selon la revendication 4 **caractérisé en ce que** l'orifice de blocage (11) et l'élément poussoir (17) sont de section transversale sensiblement parallélépipédique.

6. Dispositif selon la revendication 5 **caractérisé en ce que** l'orifice de blocage (11) et l'élément poussoir (17) ont une section transversale sensiblement rectangulaire.

7. Dispositif selon l'une des revendications précédentes **caractérisé en ce que** l'élément poussoir (17) est surmonté d'une tête (14) et est sécable de manière à séparer la tête du poussoir.

8. Dispositif selon la revendication 5 ou 6 **caractérisé en ce que** la section transversale de l'élément poussoir (17) définit des faces de grande largeur (19) et de petite largeur (20).

9. Dispositif selon la revendication 8 **caractérisé en ce que** les surfaces des faces (19, 20) sont planes ou pourvues de reliefs.

10. Dispositif selon la revendication 9 **caractérisé en ce que** les surfaces des faces (19, 20) sont pourvues de crans (20A).

## Claims

1. Surgical device intended to connect and align at least two bone parts together, comprising:
- a surgical pin (1) able to be implanted axially in the bone parts to be connected,
- a protective plug (5) provided with a through channel (9) to enable the pin (1) to be slipped in, the said plug comprising a locking orifice (11) into which a locking means (12) is intended to be introduced in order to lock the relative positions of the plug (5) and pin (1),
**characterised in that**:
- the locking orifice (11) is formed so as to open out substantially tangentially in the through channel (9) in order to form a surface S,
- the locking means (12) having a cross-section adapted so that, when it is introduced into the locking orifice (11), it can lock the plug (5) and pin (1) relatively by compressive/locking contact at the surface S.

2. Device according to Claim 1, **characterised in that** the locking orifice (11) is substantially orthogonal to the through channel (9).

3. Device according to one of the preceding claims, **characterised in that** the through channel (9) opens out in a guide groove (10), for example in a U shape, provided in the body of the plug (5) to enable a stop elbow on the pin to be produced.

4. Device according to one of the preceding claims, **characterised in that** the locking means (12) comprises a pusher element (17) with a cross-section increasing from its insertion end (18).

5. Device according to Claim 4, **characterised in that** the locking orifice (11) and the pusher element (17) have substantially parallelepipedal cross-sections.

6. Device according to Claim 5, **characterised in that** the locking orifice (11) and the pusher element (17) have substantially rectangular cross-sections.

7. Device according to one of the preceding claims, **characterised in that** the pusher element (17) is surmounted by a head (14) and is breakable so as to separate the head from the pusher.

8. Device according to Claim 5 or 6, **characterised in that** the cross-section of the pusher element (17) defines wide (19) and narrow (20) faces.

9. Device according to Claim 8, **characterised in that** the surfaces of the faces (19, 20) are flat or provided with reliefs.

10. Device according to Claim 9, **characterised in that** the surfaces of the faces (19, 20) are provided with notches (20A).

## Patentansprüche

1. Chirurgische Vorrichtung, die die Verbindung und Ausrichtung von zumindest zwei Knochenteilen untereinander gewährleisten soll, umfassend:
einen chirurgischen Stift (1), dazu geeignet, axial in die zu verbindenden Knochenteile eingesetzt zu werden,
eine Schutzkappe (5), ausgestattet mit einem Querkanal (9), zum Aufstecken auf den Stift (1), wobei vorgenannte Kappe eine Blockieröffnung (11) umfasst, in die ein Blockiermittel (12) eingeführt werden soll, um die relative Position der Kappe (5) und des Stifts (1) zu fixieren,
**dadurch gekennzeichnet, dass**:
die Blockieröffnung (11) so ausgebildet ist, dass sie im wesentlichen tangential in den Querkanal (9) mündet, so dass eine Oberfläche S gebildet wird,
das Blockiermittel (12) einen Querschnitt aufweist, der derart angepasst ist, dass er, wenn er in die Blockieröffnung (11) eingeführt wird, die Kappe (5) und den Stift (1) durch Druckkontakt/Blockierkontakt auf dem Niveau der Oberfläche S relativ blockieren kann.

2. Vorrichtung nach Anspruch 1, **dadurch gekennzeichnet, dass** die Blockieröffnung (11) etwa rechtwinklig zum Querkanal (9) ist.

3. Vorrichtung nach einem der vorhergehenden Ansprüche, **dadurch gekennzeichnet, dass** der Querkanal (9) in eine Führungsrille (10) mündet, beispielsweise U-förmig, die in dem Material der Kappe (5) ausgebildet ist, so dass eine Haltekrümmung für den Stift gebildet wird.

4. Vorrichtung nach einem der vorhergehenden Ansprüche, **dadurch gekennzeichnet, dass** das Blockiermittel (12) ein Eindrückelement (17) mit einem ausgehend von seinem Einführungsende (18) wachsenden Querschnitt umfasst.

5. Vorrichtung nach Anspruch 4, **dadurch gekennzeichnet, dass** die Blockieröffnung (11) und das Eindrückelement (17) im wesentlichen parallelepipedförmige Querschnitte haben.

6. Vorrichtung nach Anspruch 5, **dadurch gekennzeichnet, dass** die Blockieröffnung (11) und das Eindrückelement (17) einen nahezu rechteckigen Querschnitt haben.

7. Vorrichtung nach einem der vorhergehenden Ansprüche, **dadurch gekennzeichnet, dass** das Eindrückelement (17) von einem Kopf (14) bedeckt und schneidbar ist, um den Kopf vom Drücker zu trennen.

8. Vorrichtung nach Anspruch 5 oder 6, **dadurch gekennzeichnet, dass** der Querschnitt des Eindrückelements (17) Seiten großer Breite (19) und kleiner Breite (20) definiert.

9. Vorrichtung nach Anspruch 8, **dadurch gekennzeichnet, dass** die Oberflächen der Seiten (19, 20) eben oder reliefartig ausgestattet sind.

10. Vorrichtung nach Anspruch 9, **dadurch gekennzeichnet, dass** die Oberflächen der Seiten (19, 20) mit Kerben (20A) ausgestattet sind.
